(19) Europäisches Patentamt
European Patent Office
Office européen des brevets

(11) **EP 2 431 023 A1**

(12) **EUROPEAN PATENT APPLICATION**
published in accordance with Art. 153(4) EPC

(43) Date of publication:
**21.03.2012 Bulletin 2012/12**

(51) Int Cl.:
*A61K 9/127* (2006.01)    *A61K 31/05* (2006.01)
*A61K 8/14* (2006.01)     *A61P 17/18* (2006.01)
*A61P 17/10* (2006.01)    *A61Q 19/08* (2006.01)

(21) Application number: **10769229.5**

(22) Date of filing: **12.03.2010**

(86) International application number:
**PCT/CN2010/071011**

(87) International publication number:
**WO 2010/124540 (04.11.2010 Gazette 2010/44)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.04.2009 CN 200910050247**

(71) Applicant: **Shanghai Jahwa United Co., Ltd.**
**Shanghai 200082 (CN)**

(72) Inventors:
• **SHI, Qing**
**Shanghai 200082 (CN)**
• **JIN, Risheng**
**Shanghai 200082 (CN)**

• **CHEN, Jianming**
**Shanghai 200082 (CN)**
• **CHEN, Ming**
**Shanghai 200082 (CN)**
• **LI, Huiliang**
**Shanghai 200082 (CN)**
• **LV, Luo**
**Shanghai 200082 (CN)**
• **GAO, Baoan**
**Shanghai 200082 (CN)**

(74) Representative: **Jansen, Cornelis Marinus**
**VEREENIGDE**
**Johan de Wittlaan 7**
**2517 JR Den Haag (NL)**

(54) **FLEXIBLE LIPOSOME OF RESVERATROL AND PREPARATION METHOD THEREOF**

(57)    The present invention relates to a resveratrol flexible liposome, comprising the following components with respect to the weight of the liposome: between 0.1 and 10% of resveratrol, between 0.5 and 10% of phospholipid and between 0.1 and 30% of softener. In addition, the present invention also relates to methods for preparing the resveratrol flexible liposome. The resveratrol flexible liposome of the present invention is highly flexible and has high penetrability, thus resulting in greatly increased transdermal absorption of the active ingredient.

Curves of the accumulated amount of penetration between two flexible liposomes

Fig. 1

**EP 2 431 023 A1**

**Description**

Technical Field

The present invention relates to the cosmetic field, especially to a resveratrol flexible liposome and the preparation method thereof.

Background

Resveratrol with its chemical name being 3,4,5-trihydroxystilbene, mainly exists in plants such as grape, polygonum cuspidatum, peanut, clover, mulberry, gnetum montanum, maackia amurensis, etc. It is a non-flavonoid polyphenolic compound having stilbene structure and has two isomers, i.e., cis and trans isomers. In nature, it mainly exists as the trans form.

It has been proven that resveratrol has good anti-aging effects and is identified as one of the most effective anti-aging substance. The mechanism of action consists in its abilities to scavenge the free radical in vivo, activate the function factors in vivo, promote and keep the proper metabolism in vivo, as well as harmonize the internal and external secretion. The free radical-scavenging effect of resveratrol makes it a good natural antioxidant and suitable to be used as anti-aging agent. In addition, the anti-inflammatory and bactericidal effects of resveratrol are highly suitable for treating and getting rid of skin acne and herpes etc. Additionally, resveratrol also has the effects of keeping the skin moisture and protecting the skin from ultraviolet radiation. Therefore, resveratrol has a wide range of applications in the cosmetic field. However, resveratrol is slightly soluble in water and has a poor biological compatibility, thus it is difficult to permeate the skin and is not suitable to make into hydrophilic cosmetics. In addition, resveratrol also undergoes photolysis extremely easily. Therefore, the above properties of resveratrol limit its application in the cosmetic field.

Based on long-term research, the inventor has found that it is possible to increase the skin penetration of resveratrol thus, to increase the skin absorption of resveratrol by making resveratrol into a flexible liposome. Meanwhile, making resveratrol into a flexible liposome can also reduce the photolysis of resveratrol and increase its stability.

Invention Details

The technical problem underlying the invention is to provide a resveratrol flexible liposome with good transdermal absorption property.

The resveratrol flexible liposome of the present invention comprises the following components with respect to the weight of the liposome: between 0.1 and 10% of resveratrol, between 0.5 and 10% of phospholipid and between 0.1 and 30% of softener.

Preferably, resveratrol is present in an amount of between 0.1 and 7% by weight, the phospholipid is present in an amount of between 0.5 and 8% by weight, and the softener is present in an amount of between 0.1 and 20% by weight.

More preferably, resveratrol is present in an amount of between 0.5 and 5% by weight, the phospholipid is present in an amount of between 1 and 5% by weight, and the softener is present in an amount of between 1 and 10% by weight.

The resveratrol flexible liposome of the present invention may further comprise a suitable amount of antioxidant, preservative and stabilizer. One skilled in the art can easily determine said "suitable amount" according to the specific kinds of the selected antioxidant, preservative and stabilizer.

Preferably, the stabilizer is present in an amount of greater than 0 and less than or equal to 15% by weight, the antioxidant is present in an amount of between 0.005 and 0.5% by weight, and the preservative is present in an amount of between 0.005 and 0.5% by weight.

More preferably, the stabilizer is present in an amount of greater than 0 and less than or equal to 10% by weight.

The phospholipid used in the resveratrol flexible liposome of the present invention may be one or more selected from the group consisting of soybean lecithin, egg yolk lecithin, distearoyl phosphatidylcholine, dipalmitoyl phosphatidylcholine, and dimyristoyl phosphatidylcholine. Preferably, the phospholipid used in the resveratrol flexible liposome of the present invention is one or more selected from the group consisting of soybean lecithin and egg yolk lecithin.

The softener may be one or more selected from the group consisting of Tween 80, cholic acid, sodium cholate, sodium deoxycholate, Span 80, polyoxyethylene (2) cetyl ether, propylene glycol and absolute ethanol. Preferably, the softener is one or more selected from the group consisting of sodium cholate, Tween 80, propylene glycol and absolute ethanol.

The stabilizer may be one or more selected from the group consisting of dicetyl phosphate, stigmasterol, sitosterol, cermaide, oleic acid, sodium oleate, glycerylmonooleate, glyceryl caprylate, glyceryl caprate, caprylic/capric triglyceride and poloxamer. Preferably, the stabilizer is one or more selected from the group consisting of dicetyl phosphate, caprylic/capric triglyceride and poloxamer.

The antioxidant may be one or more selected from the group consisting of vitamin E, butylated hydroxyanisole, butylated hydroxytoluene, propyl gallate, vitamin C, sodium pyrosulfite and ethylenediamine tetraacetic acid. Preferably, the antioxidant is one or more selected from the group consisting of vitamin E, vitamin C, and sodium pyrosulfite.

The preservative may be one or more selected from the group consisting of ethyl p-hydroxybenzoate, propyl p-hydroxybenzoate and benzalkonium chloride. Preferably, the preservative is one or more selected from the group consisting of ethyl p-hydroxybenzoate and propyl p-hydroxybenzoate.

The present invention also provides a method for preparing a resveratrol flexible liposome, comprising the following steps in turn:

    (1) Preparation of a lipid solution: mixing the pharmaceutical active ingredient, i.e. resveratrol, phospholipid and a lipid soluble softener, adding the mixture into a suitable organic solvent, and dissolving the mixture by heating at a temperature of 50-100°C;
    (2) preparation of a lipid hydrated suspension: placing the lipid solution in a rotatory evaporator, and volatilizing the organic solvent via rotary film evaporation at a temperature of 50-85°C until a lipid film is formed on the bottom of the evaporator, then adding water for hydration to form a lipid hydrated suspension;
    (3) subjecting the lipid hydrated suspension to one or more technical processes selected from homogenization, ultrasonic treatment and shearing to obtain a resveratrol flexible liposome.

The above preparation method can further comprise: in preparation of the lipid hydrated suspension of step (2), after the formation of the lipid film on the bottom of the evaporator, adding an aqueous solution comprising a water soluble softener for hydration to form the lipid hydrated suspension.

The present invention also provides another method for preparing a resveratrol flexible liposome, comprising the following steps in turn:

    (1) preparation of a lipid solution: mixing the pharmaceutical active ingredient, i.e. resveratrol and phospholipid, adding the mixture into a suitable organic solvent, and dissolving the mixture by heating at a temperature of 50-100°C;
    (2) preparation of a lipid hydrated suspension: placing the lipid solution in a rotatory evaporator, and volatilizing the organic solvent via rotary film evaporation at a temperature of 50-85°C until a lipid film is formed on the bottom of the evaporator, then adding an aqueous solution comprising a water soluble softener for hydration to form a lipid hydrated suspension;
    (3) subjecting the lipid hydrated suspension to one or more common technical processes selected from homogenization, ultrasonic treatment and shearing to obtain a resveratrol flexible liposome.

The present invention also provides another method for preparing a resveratrol flexible liposome, comprising the following steps in turn:

    (1) preparation of a lipid solution: mixing the pharmaceutical active ingredient, i.e. resveratrol, phospholipid and a lipid soluble softener, and melting the mixture by heating at a temperature of 50-100°C;

    (2) preparation of a lipid hydrated suspension: dispersing the lipid solution directly into the water of a temperature of 50-85°C to form a lipid hydrated suspension;

    (3) subjecting the lipid hydrated suspension to one or more technical processes selected from homogenization, ultrasonic treatment and shearing to obtain a resveratrol flexible liposome.

The above preparation method can further comprise: in preparation of the lipid hydrated suspension of step (2), adding the lipid solution directly into an aqueous solution comprising a water soluble softener of a temperature of 50-85°C for hydration to form the lipid hydrated suspension.

The present invention also provides another method for preparing a resveratrol flexible liposome, comprising the following steps in turn:

    (1) preparation of a lipid solution: mixing the pharmaceutical active ingredient, i.e. resveratrol and phospholipid, and melting the mixture by heating at a temperature of 50-100°C;
    (2) preparation of a lipid hydrated suspension: dispersing the lipid solution directly into an aqueous solution comprising a water soluble softener of a temperature of 50-85°C to form a lipid hydrated suspension;
    (3) subjecting the lipid hydrated suspension to one or more technical processes selected from homogenization, ultrasonic treatment and shearing to obtain a resveratrol flexible liposome.

The term "suitable organic solvent" used in the above preparation methods means any organic solvent capable of dissolving the mixture obtained in step (1). One skilled in the art can determine the specific organic solvent according to the substances used in the step (1). Said "suitable organic solvent" is preferably one or more selected from the group consisting of dichloromethane, chloroform, diethyl ether and methanol.

All the above mentioned methods for preparing the resveratrol flexible liposome can further comprise the following steps:

in preparation of the lipid solution in step (1), one or more lipid soluble components selected from the stabilizer, antioxidant and preservative are mixed with resveratrol and phospholipid, or with resveratrol, phospholipid and a lipid soluble softener to obtain the lipid solution; and/or

in preparation of the lipid hydrated suspension in step (2), one or more water soluble components selected from the stabilizer, antioxidant and preservative are added with the water or an aqueous solution comprising a water soluble softener to obtain the lipid hydrated suspension.

The above steps which the methods can further comprise means that the lipid soluble component(s) is/are added during the preparation of the lipid solution in step (1), and the water soluble component(s) is/are added during the preparation of the lipid hydrated suspension of step (2), depending on the solubility of the selected stabilizer, antioxidant and preservative.

The resveratrol flexible liposome of the present invention can be used as a part of a cosmetic formulation for the preparation of the cosmetic formulation in any suitable form such as emulsion, cream, oil, lotion, rod, gel and aerosol, and applied to the skin surface via a common method.

In addition to the advantages of a conventional resveratrol liposome, the resveratrol flexible liposome of the present invention is also highly flexible and has high penetrability, thus resulting in increased transdermal absorption of the active ingredient.

It has been proven that the resveratrol flexible liposome of the present invention has a greater deformability than a conventional resveratrol liposome by 5 orders of magnitude. For example, the resveratrol flexible liposome having a mean particle size of 500nm can pass through a pore having a size of 1/5 of the liposome, with the diameter of the liposome before and after the process almost remaining unchanged. Such property of the resveratrol flexible liposome is derived from the heterogeneity of the lipid membrane. This is because the softener with surface active effect is present in the lipid membrane, and the softener aggregates easily at the high winding area. Therefore, during the deformation process, the surface energy (elastic energy) required to be overcome is greatly reduced, which results in high deformability of the resveratrol flexible liposome.

Furthermore, the resveratrol flexible liposome of the present invention also has high hydrophilicity, which makes it possible to penetrate the skin along the hydration gradient. Since the resveratrol flexible liposome has high deformability, when subjected to sufficiently high stress, it can freely pass through the pore passage several times smaller than itself, thereby resulting in high penetration. When the resveratrol flexible liposome suspension is applied to the skin surface, with the continuous evaporation of the solvent, the high hydrophilicity allows it to penetrate into the skin by means of the hydration gradient (the skin's own unique property) gradually increasing from the surface of the skin to deep tissue. Its high deformability can ensure a successful penetration. However, the conventional resveratrol liposome still remains on the skin surface until dehydration and fusion due to lack of deformability.

Brief Description of the Drawings

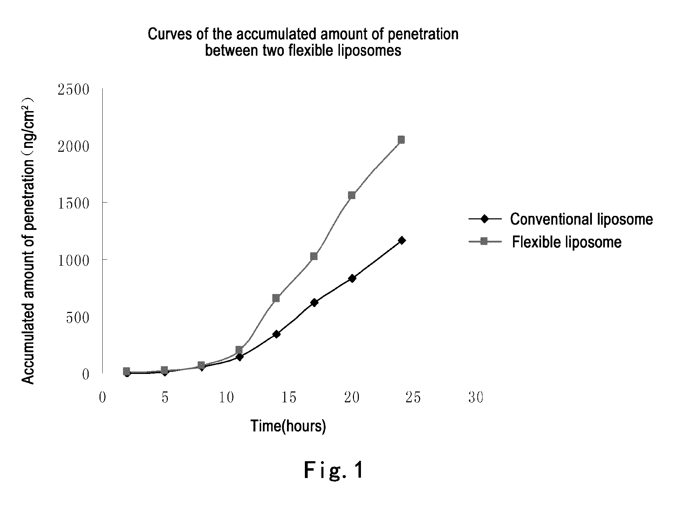

Fig. 1 shows curves of the accumulated amount of penetration per unit area, of the flexible liposome of the present invention and of the conventional liposome (X, n=6).

Embodiment of the Present Invention

Further explanation of this invention with reference to Examples is given below. The Examples shall not be construed as a limitation on the scope of the invention in any way.

Example 1

1.2g resveratrol, 3g soybean lecithin and 0.01g vitamin E were mixed in an eggplant shaped bottle and dissolved with 30ml chloroform by heating at 45°C to obtain a lipid solution; then the organic solvent was volatilized via rotation at 55°C to form a lipid film; then 94.5g distilled water of 60°C containing 0.2g sodium cholate, 1.8g propylene glycol and 0.015g propyl p-hydroxybenzoate was added for hydrateion to form a lipid hydrated suspension; said lipid hydrated suspension was subjected to high pressure homogeneous emulsification to obtain the resveratrol flexible liposome.

Example 2

0.3g resveratrol, 0.8g soybean lecithin, 0.01 butylated hydroxyanisole and 1.0g glycerylmonooleate were mixed in an eggplant shaped bottle and melted by heating at 100°C to obtain a lipid solution; then the lipid solution was dispersed in 97.4g distilled water of 80°C containing 0.4g Tween 80, 0.015g sodium pyrosulfite and 0.01g ethyl p-hydroxybenzoate to form a lipid hydrated suspension; said lipid hydrated suspension was subjected to high pressure homogeneous emulsification to obtain the resveratrol flexible liposome.

Example 3

1.2g resveratrol, 3g soybean lecithin, 12g propylene glycol, 10g caprylic/capric triglyceride, 3g dicetyl phosphate and 0.02g butylated hydroxytoluene were mixed in an eggplant shaped bottle and melted by heating at 95°C to obtain a lipid solution; then the lipid solution was dispersed in 58.8g distilled water of 80°C containing 12g Tween 80, 0.008g ethylenediamine tetraacetic acid and 0.005g benzalkonium chloride to form a lipid hydrated suspension; said lipid hydrated suspension was subjected to high pressure homogeneous emulsification to obtain the resveratrol flexible liposome.

Example 4

2g resveratrol, 4g egg yolk lecithin, 3g stigmasterol and 0.006g propyl gallate were mixed and dissolved with 8ml absolute ethanol by heating at 60°C to obtain a lipid solution; then the lipid solution was dispersed in 82.7g distilled water of 60°C containing 0.2g sodium cholate, 0.8g propylene glycol, 0.015g ethyl p-hydroxybenzoate and 0.01g propyl p-hydroxybenzoate to form a lipid hydrated suspension; said lipid hydrated suspension was subjected to shear and high pressure homogeneous emulsification to obtain the resveratrol flexible liposome.

Example 5

3g resveratrol, 3g soybean lecithin, 2g egg yolk lecithin, 4g Span 80, 2.7g polyoxyethylene (2) cetyl ether, 0.3g cholic acid, 6g sitosterol and 0.008g vitamin E were mixed and melted by heating at 78°C, then 78.96g distilled water of 60°C containing 0.03g sodium pyrosulfite and 0.015g ethyl p-hydroxybenzoate was added for hydration to form a lipid hydrated suspension; said lipid hydrated suspension was subjected to ultrasonic emulsification to obtain the resveratrol flexible liposome.

Example 6

4g resveratrol, 1.6g distearoyl phosphatidylcholine, 4g dipalmitoyl phosphatidylcholine, 0.1g cholic acid, 3.9g absolute ethanol and 9g glyceryl caprylate were mixed in an eggplant shaped bottle and melted by heating at 85°C to obtain a lipid solution; then the lipid solution was dispersed in 71.36g distilled water of 80°C containing 6g Tween 80, 0.03g sodium pyrosulfite and 0.005g benzalkonium chloride to form the lipid hydrated suspension; said lipid hydrated suspension was subjected to shear emulsification to obtain the resveratrol flexible liposome.

Example 7

4.7g resveratrol, 9g dimyristoyl phosphatidylcholine, 6g cholic acid, 4g glyceryl caprate, 3g dicetyl phosphate and 0.015g vitamin E were mixed and dissolved with 5ml absolute ethanol by heating at 75°C to obtain a lipid solution; then the lipid solution was dispersed in 70.27g distilled water of 75°C containing 0.015g propyl p-hydroxybenzoate for hydration to form a lipid hydrated suspension; said lipid hydrated suspension was subjected to high pressure homogeneous emulsification to obtain the resveratrol flexible liposome.

Example 8

6g resveratrol, 7.2g egg yolk lecithin, 3g sitosterol, 3.8g cermaide and 1.2g oleic acid were mixed and dissolved with 10ml absolute ethanol by heating at 80°C to obtain a lipid solution; then the lipid solution was dispersed in 73.8g distilled water of 80°C containing 1.3g sodium deoxycholate, 3.7g propylene glycol, 0.005g vitamin C, 0.04g sodium pyrosulfite and 0.006g benzalkonium chloride for hydration to form a lipid hydrated suspension; said lipid hydrated suspension was subjected to high pressure homogeneous emulsification to obtain the resveratrol flexible liposome.

Example 9

7g resveratrol, 8g soybean lecithin, 4g cholic acid, 4g polyoxyethylene (2) cetyl ether, 12g glyceryl caprylate and 3g poloxamer were mixed and melted by heating at 70°C to obtain a lipid solution; then the lipid solution was dispersed in 53.97g distilled water of 70°C containing 8g Tween 80, 0.015g vitamin C and 0.01g ethyl p-hydroxybenzoate to form a lipid hydrated suspension; said lipid hydrated suspension was subjected to shear emulsification and ultrasonic emulsification to obtain the resveratrol flexible liposome.

Example 10

9.5g resveratrol, 8g soybean lecithin, 2g dipalmitoyl phosphatidylcholine, 13g caprylic/capric triglyceride, 4g oleic acid and 0.05g butylated hydroxytoluene were mixed and melted by heating at 90°C to obtain a lipid solution; then the lipid solution was dispersed in 49.48g distilled water of 70°C containing 0.2g sodium cholate, 3.8g Tween 80, 10g propylene glycol, 0.01g ethyl p-hydroxybenzoate and 0.015g propyl p-hydroxybenzoate to form a lipid hydrated suspension; said lipid hydrated suspension was subjected to shear emulsification to obtain the resveratrol flexible liposome.

Example 11

1.5g resveratrol, 4.8g egg yolk lecithin, 2g polyoxyethylene (2) cetyl ether and 4g dicetyl phosphate were mixed in an eggplant shaped bottle and dissolved with 25ml dichloromethane by heating at 60°C to obtain a lipid solution; then the organic solvent was volatilized via rotation at 60°C to form a lipid film; 85.67g distilled water of 60°C containing 2g sodium cholate, 0.03g sodium pyrosulfite and 0.008g benzalkonium chloride was added for hydration to form a lipid hydrated suspension; said lipid hydrated suspension was subjected to high pressure homogeneous emulsification to obtain the resveratrol flexible liposome.

Example 12

1g resveratrol and 2.5g egg yolk lecithin were mixed in an eggplant shaped bottle and dissolved with 5ml absolute ethanol by heating at 58°C to obtain a lipid solution; then 89.47g distilled water of 58°C containing 3g Tween 80, 1g sodium cholate, 0.02g vitamin C and 0.01g ethyl p-hydroxybenzoate was added for hydration to form a lipid hydrated suspension; said lipid hydrated suspension was subjected to high pressure homogeneous emulsification to obtain the resveratrol flexible liposome.

Example 13

2g resveratrol, 3.3g soybean lecithin and 0.05g caprylic/capric triglyceride were mixed in an eggplant shaped bottle and dissolved with 20ml chloroform by heating at 55°C to obtain a lipid solution; then the organic solvent was volatilized via rotation at 60°C to form a lipid film; 92.37g distilled water of 55°C containing 0.1g sodium cholate, 1.1g propylene glycol, 0.02g vitamin C and 0.015g ethyl p-hydroxybenzoate was added t for hydration to form a lipid hydrated suspension; said lipid hydrated suspension was subjected to high pressure homogeneous emulsification to obtain the resveratrol flexible liposome.

Example 14

3g resveratrol, 4.5g soybean lecithin, 3g dicetyl phosphate and 4g caprylic/capric triglyceride were mixed and dissolved with 6ml absolute ethanol by heating at 80°C to obtain a lipid solution; then the lipid solution was dispersed in 79.47g distilled water of 80°C containing 4g Tween 80, 2g propylene glycol, 0.03g sodium pyrosulfite and 0.008g benzalkonium chloride to form the lipid hydrated suspension; said lipid hydrated suspension was subjected to high pressure homogeneous emulsification to obtain the resveratrol flexible liposome.

Example 15

4.5g resveratrol, 5g soybean lecithin, 3g dicetyl phosphate, 5g caprylic/capric triglyceride and 0.02g vitamin E were mixed and dissolved with 7ml absolute ethanol by heating at 75°C to obtain the lipid solution; then the lipid solution was dispersed in 73.46g distilled water of 75°C containing 2g propylene glycol, 0.01g ethyl p-hydroxybenzoate and 0.015g propyl p-hydroxybenzoate for hydration to form a lipid hydrated suspension; said lipid hydrated suspension was subjected to high speed shear emulsification to obtain the resveratrol flexible liposome.

Example 16

1.2g resveratrol, 3.5g soybean lecithin, 3g dicetyl phosphate, 2g caprylic/capric triglyceride and 0.008g butylated hydroxytoluene were mixed and dissolved with 6ml absolute ethanol by heating at 68°C to obtain a lipid solution; then the lipid solution was dispersed in 80.3g distilled water of 70 °C containing 7g Tween 80, 3g propylene glycol, 0.04g benzalkonium chloride for hydration to form a lipid hydrated suspension; said lipid hydrated suspension was subjected to ultrasonic emulsification to obtain the resveratrol flexible liposome.

Example 17

1.8g resveratrol, 4g soybean lecithin, 3g dicetyl phosphate and 4g poloxamer were mixed and melted by heating at 80°C to obtain a lipid solution; then the lipid solution was dispersed in 81.2g distilled water of 80°C containing 1g sodium cholate, 6g propylene glycol, 0.04g sodium pyrosulfite and 0.01g ethyl p-hydroxybenzoate for hydration to form a lipid hydrated suspension; said lipid hydrated suspension was subjected to high pressure homogeneous emulsification to obtain the resveratrol flexible liposome.

Example 18

1g resveratrol, 2.5g soybean lecithin, 4g dicetyl phosphate and 0.02g vitamin E were mixed in an eggplant shaped bottle and dissolved with 22ml chloroform by heating at 55°C to obtain a lipid solution; then the organic solvent was volatilized via rotation at 60°C to form a lipid film; 87.42g distilled water of 60°C containing 2g sodium cholate, 3g propylene glycol, 0.04g sodium pyrosulfite and 0.02g propyl p-hydroxybenzoate was added for hydration to form a lipid hydrated suspension; said lipid hydrated suspension was subjected to high pressure homogeneous emulsification to obtain the resveratrol flexible liposome.

Example 19

2g resveratrol, 2g soybean lecithin, 2g egg yolk lecithin, 3g polyoxyethylene (2) cetyl ether, 3g dicetyl phosphate and 0.05g propyl gallate were mixed in an eggplant shaped bottle and dissolved with 5ml absolute ethanol by heating at 80°C to obtain a lipid solution; then the organic solvent was volatilized via rotation at 80°C to form a lipid film; 82.9g distilled water of 80°C containing 5g Tween 80 and 0.04g benzalkonium chloride was added for hydration to form a lipid hydrated suspension; said lipid hydrated suspension was subjected to high pressure homogeneous emulsification to obtain the resveratrol flexible liposome.

Example 20

5g resveratrol, 5g egg yolk lecithin, 7g Span 80, 2g dicetyl phosphate and 8g caprylic/capric triglyceride were mixed in an eggplant shaped bottle and dissolved with 35ml chloroform by heating at 60 °C to obtain a lipid solution; then the organic solvent was volatilized via rotation at 55°C to form a lipid film; 69.96g distilled water of 55°C containing 3g sodium deoxycholate, 0.02g sodium pyrosulfite and 0.02g propyl p-hydroxybenzoate was added for hydration to form a lipid hydrated suspension; said lipid hydrated suspension was subjected to high pressure homogeneous emulsification to obtain the resveratrol flexible liposome.

Example 21

1.5g resveratrol and 4.2g soybean lecithin were mixed in an eggplant shaped bottle and dissolved with 22ml dichloromethane by heating at 60°C to obtain a lipid solution; then the organic solvent is volatilized via rotation at 60°C to form a lipid film; 86.23g distilled water of 70°C containing 2g sodium cholate, 6g propylene glycol, 0.05g vitamin C and 0.02g ethyl p-hydroxybenzoate was added for hydration to form a lipid hydrated suspension; said lipid hydrated suspension was subjected to high pressure homogeneous emulsification to obtain the resveratrol flexible liposome.

Comparative Examples

In order to better understand the advantages of the resveratrol flexible liposome of the present invention, a comparison between the resveratrol flexible liposome of the present invention and the conventional resveratrol liposome was conducted by the following experiments using in vitro transdermal test and deformability test.

The resveratrol flexible liposome prepared by Example 1 was used as the test sample.

The conventional resveratrol liposome was prepared by the following method: 1.2g resveratrol, 3g soybean lecithin and 0.2g cholesterol were mixed in an eggplant shaped bottle and dissolved with a suitable amount of chloroform by heating at 45°C to obtain a lipid solution; then the organic solvent was volatilized via rotation at 55°C to form a lipid film; 95.6g distilled water of 60°C was added for hydration to form a lipid hydrated suspension; said lipid hydrated suspension was subjected to high pressure homogeneous emulsification to obtain the conventional resveratrol liposome as a control.

I. In vitro transdermal test

1. Material and method

1.1. The preparation of the in vitro mouse skin

6 female naked mice (purchased from the experimental animal centre of the Second Military Medical University) were killed by cervical dislocation, the back skin was taken, from which the subcutaneous tissue was removed, and was washed with normal saline before being frozen until use.

1.2. Diffuse experimental device and method

By using modified Franz diffusion cells, the mouse skin was fixed between the supply chamber and the acceptance chamber with stratum corneum facing the supply chamber and dermis facing the acceptance chamber. The dermis was kept close contact with the acceptance solution that is normal saline. The contact area between the skin and the acceptance solution was $1.0cm^2$, the volume of the acceptance solution was 4.2ml, and temperature of the thermostatic water bath is 32°C. Each 1ml of the sample of the flexible liposome and the conventional liposome was added to different supply chambers and evenly applied to the surface of the stratum corneum of the mouse skin. Under continuous agitation, 1ml acceptance solution was taken at 2h, 5h, 8h, 11h, 14h, 17h, 20h and 24h after application of the liposomes, and filtrated through $0.45\mu m$ millipore filter, then stored at 4°C until the determination of the concentration of resveratrol. At the same time, the same amount of fresh blank acceptance solution at the same temperature was supplemented. The experimental process was conducted under protection from light in order to avoid the occurrence of the photolysis reaction of resveratrol under the experiment condition.

1.3. The determination of the accumulated amount of penetration per unit area

Liquid phase condition: Agilent 1100 liquid chromatography, Agilent G1314A VWD detector, Agilent G1311A Type four-pumps, Agilent G1314A VWD detector, Agilent LC1100 chromatographic data work station, Diamonsil C18 analytical column ($5\mu$, 4.6mmx250mm), acetonitrile-0.2% glacial acetic acid (35:65) as the mobile phase, flow rate 1.0ml/min, detection wavelength 306nm, and column temperature 25°C.

Test method: 1ml of acceptance solution was filtrated through $0.45\mu m$ millipore filter, and subsequent filtrate was used as sample solution. A suitable amount of resveratrol (control) was weighed precisely and placed in a brown volumetric flask, then methanol was added to achieve a concentration of $50\mu g$ resveratrol per 1 ml solution. 1.0ml of solution was transferred precisely and placed in a brown volumetric flask, then the normal saline was added to the calibration and homogenously mixed. The obtained solution was used as a control solution. Each $100\mu l$ of the sample solution and the control solution was transferred precisely and injected into liquid chromatograph. The concentration of the resveratrol ($Cn$) was calculated by external standard method.

Calculation formula of the accumulated amount of penetration per unit area:

$$Q_n = \left( C_n V + \sum_{i=1}^{n-1} C_i V_i \right)/s$$

wherein $Q_n$ is the accumulated amount of penetration per unit area at time point n; $C_n$ is the concentration measured at time point n; $C_i$ is the concentration measured at time point n-1; V is the volume of the acceptance solution; $V_i$ is the volume of the sample; and S is the diffusion area.

1.4. Statistical method

The accumulated amount of penetration of the resveratrol ($ng/cm^2$) at different time point was calculated for the acceptance solution. All the obtained results were expressed in mean $\pm$ standard deviation ($\bar{x} \pm SD$). The two groups of the results were compared by Student test.

2. Test results

2.1 The stability of resveratrol in the normal saline

A suitable amount of resveratrol was weighed and placed in a brown volumetric flask, firstly dissolved in small amount of methanol and then diluted with the normal saline to the calibration. The stability was examined by placing the sample in a water bath of 32°C under protection from light. The result showed that the resveratrol is still quite stable after 24 hours under the condition of placing the sample in a water bath of 32°C and protecting from light. The results are shown in table 1 (comparison of the peak area).

Table 1. The stability test of resveratrol in the normal saline

| Time(h) | 0 | 3 | 6 | 18 | 24 |
|---|---|---|---|---|---|
| peak area | 378.9 | 363.2 | 376.5 | 377.7 | 369.9 |

2.2 Accumulated amount of penetration

During the 24h of penetration, there was a significant difference between the accumulated amount of penetration of the resveratrol flexible liposome and of the conventional resveratrol liposome against the in vitro naked mouse skin. The accumulated amount of penetration of the resveratrol flexible liposome against the in vitro naked mouse skin is greater than that of the conventional resveratrol liposome ($P<0.05$). The results are shown in table 2 and Fig. 1.

Table 2. The accumulated amounts of penetration of the two resveratrol liposomes against the in vitro naked mouse skin ($ng/cm^2$, n=6)

| Penetration time(h) | conventional liposome | flexible liposome |
|---|---|---|
| 2 | $3.87 \pm 1.77$ | $9.25 \pm 2.76$ |
| 5 | $12.73 \pm 2.12$ | $21.55 \pm 2.85$ |
| 8 | $52.48 \pm 9.11$ | $68.93 \pm 8.69$ |
| 11 | $139.80 \pm 20.71$ | $205.13 \pm 32.95$ |
| 14 | $345.35 \pm 60.61$ | $655.4 \pm 110.92$ |
| 17 | $618.38 \pm 90.03$ | $1017.48 \pm 132.58$ |
| 20 | $829.13 \pm 74.1$ | $1553.87 \pm 170.78$ |
| 24 | $1165.6 \pm 131.2$ | $2039.08 \pm 323.19$ |

3. Result

The in vitro transdermal test has shown that there was a significant difference between the accumulated amount of penetration of the resveratrol flexible liposome against the in vitro naked mouse skin and that of the conventional resveratrol liposome, and that the accumulated amount of penetration of the resveratrol flexible liposome against the in vitro naked mouse skin is greater than that of conventional resveratrol liposome.

II. Deformability test

1. Method

The comparison between the resveratrol flexible liposome and the conventional liposome in terms of their deformability to pass through the millipore filter with a pore diameter of $0.20\mu m$ were conducted under an external pressure of 0.2MPa. First, the rate (V water), at which the distilled water passes through the millipore filter with a pore diameter of $0.20\mu m$, was measured, then the rates ($V_{transfersomes}$), at which the resveratrol flexible liposome and the conventional liposome pass through the millipore filter with a pore diameter of 0.20μm were measured respectively. The relative penetration rate was calculated by the following formula:

$$P = V_{\text{transfersomes}}/V_{\text{water}} \times 100\ \%.$$

2. Result

The resveratrol flexible liposome of the present invention has a p value of 88% while the conventional resveratrol liposome has a p value of 56%, under an external pressure of 0.2MPa.

3. Conclusion

This test has shown that the resveratrol flexible liposome of the present invention has a better deformability than the conventional resveratrol liposome.

**Claims**

1. A resveratrol flexible liposome, comprising the following components with respect to the weight of the liposome: between 0.1 and 10% of resveratrol, between 0.5 and 10% of phospholipid and between 0.1 and 30% of a softener.

2. The resveratrol flexible liposome according to claim 1, wherein the resveratrol is present in an amount of between 0.1 and 7% by weight, the phospholipid is present in an amount of between 0.5 and 8% by weight, and the softener is present in an amount of between 0.1 and 20% by weight.

3. The resveratrol flexible liposome according to claim 2, wherein the resveratrol is present in an amount of between 0.5 and 5% by weight, the phospholipid is present in an amount of between 1 and 5% by weight, and the softener is present in an amount of between 1 and 10% by weight.

4. The resveratrol flexible liposome according to claim 1, further comprising a suitable amount of stabilizer, antioxidant and preservative.

5. The resveratrol flexible liposome according to claim 4, wherein the stabilizer is present in an amount of greater than 0 and less than or equal to 15% by weight, the antioxidant is present in an amount of between 0.005 and 0.5 % by weight, and the preservative is present in an amount of between 0.005 and 0.5% by weight.

6. The resveratrol flexible liposome according to claim 5, wherein the stabilizer is present in an amount of greater than 0 and less than or equal to 10% by weight.

7. The resveratrol flexible liposome according to claim 4, wherein:

   the phospholipid is one or more selected from the group consisting of soybean lecithin, egg yolk lecithin, distearoyl phosphatidylcholine, dipalmitoyl phosphatidylcholine, and dimyristoyl phosphatidylcholine;
   the softener is one or more selected from the group consisting of Tween 80, cholic acid, sodium cholate, sodium deoxycholate, Span 80, polyoxyethylene (2) cetyl ether, propylene glycol and absolute ethanol;
   the stabilizer is one or more selected from the group consisting of dicetyl phosphate, stigmasterol, sitosterol, cholesterol, cermaide, oleic acid, sodium oleate, glyceryl monooleate, glyceryl caprylate, glyceryl caprate, caprylic/capric triglyceride and poloxamer;
   the antioxidant is one or more selected from the group consisting of vitamin E, butylated hydroxyanisole, butylated hydroxytoluene, propyl gallate, vitamin C, sodium pyrosulfite and ethylenediamine tetraacetic acid; and
   the preservative is one or more selected from the group consisting of ethyl p-hydroxybenzoate, propyl p-hydroxybenzoate and benzalkonium chloride.

8. The resveratrol flexible liposome according to claim 7, wherein:

   the phospholipid is one or more selected from the group consisting of soybean lecithin and egg yolk lecithin;

the softener is one or more selected from the group consisting of Tween 80, sodium cholate, propylene glycol and absolute ethanol;

the stabilizer is one or more selected from the group consisting of dicetyl phosphate, caprylic/capric triglyceride and poloxamer;

the antioxidant is one or more selected from the group consisting of vitamin E, vitamin C, and sodium pyrosulfite; and

the preservative is one or more selected from the group consisting of ethyl p-hydroxybenzoate, and propyl p-hydroxybenzoate.

9. A method for preparing the resveratrol flexible liposome according to claim 1, comprising the following steps in turn:

(1) preparation of a lipid solution: mixing resveratrol, phospholipid and a lipid soluble softener, adding the mixture into a suitable organic solvent, and dissolving the mixture by heating at a temperature of 50-100°C;
(2) preparation of a lipid hydrated suspension: placing the lipid solution in a rotatory evaporator, and volatilizing the organic solvent via rotary film evaporation at a temperature of 50-85°C until a lipid film is formed on the bottom of the evaporator, then adding water for hydration to form a lipid hydrated suspension;
(3) preparation of the flexible liposome: subjecting the lipid hydrated suspension to one or more technical processes selected from homogenization, ultrasonic treatment and shearing to obtain the resveratrol flexible liposome.

10. The method according to claim 9, wherein in preparation of the lipid hydrated suspension of step (2), after the formation of the lipid film on the bottom of the evaporator, adding an aqueous solution comprising a water soluble softener for hydration to form the lipid hydrated suspension.

11. A method for preparing the resveratrol flexible liposome according to claim 1, comprising the following steps in turn:

(1) preparation of a lipid solution: mixing resveratrol and phospholipid, adding mixture into a suitable organic solvent, and dissolving the mixture by heating a temperature of 50-100°C;
(2) preparation of a lipid hydrated suspension: placing the lipid solution in a rotatory evaporator, and volatilizing the organic solvent via rotary film evaporation at a temperature of 50-85°C until a lipid film is formed on the bottom of the evaporator, then adding an aqueous solution comprising a water soluble softener for hydration to form a lipid hydrated suspension;
(3) preparation of the flexible liposome: subjecting the lipid hydrated suspension to one or more technical processes selected from homogenization, ultrasonic treatment and shearing to obtain the resveratrol flexible liposome.

12. A method for preparing the resveratrol flexible liposome according to claim 1, comprising the following steps in turn:

(1) preparation of a lipid solution: mixing resveratrol, phospholipid and a lipid soluble softener, and melting the mixture by heating at a temperature of 50-100°C;
(2) preparation of a lipid hydrated suspension: dispersing the lipid solution directly into the water of a temperature of 50-85°C to form a lipid hydrated suspension;
(3) preparation of the flexible liposome: subjecting the lipid hydrated suspension to one or more technical processes selected from homogenization, ultrasonic treatment and shearing to obtain the resveratrol flexible liposome.

13. The method according to claim 12, wherein in preparation of the lipid hydrated suspension of step (2), adding the lipid solution directly into an aqueous solution comprising a water soluble softener of a temperature of 50-85°C for hydration to form the lipid hydrated suspension.

14. A method for preparing the resveratrol flexible liposome according to claim 1, comprising the following steps in turn:

(1) preparation of a lipid solution: mixing resveratrol and phospholipid, and melting the mixture by heating at a temperature of 50-100°C;
(2) preparation of a lipid hydrated suspension: dispersing the lipid solution directly into an aqueous solution comprising a water soluble softener of a temperature of 50-85°C to form a lipid hydrated suspension;
(3) preparation of the flexible liposome: subjecting the lipid hydrated suspension to one or more technical processes selected from homogenization, ultrasonic treatment and shearing to obtain the resveratrol flexible liposome.

15. The method according to any one of claims 9-14, wherein:

in preparation of the lipid solution in step (1), a lipid soluble stabilizer and/or an antioxidant and/or a preservative are mixed with the resveratrol and phospholipid to obtain the lipid solution; or with the resveratrol, phospholipid and a lipid soluble softener to obtain the lipid solution; and/or
in preparation of the lipid hydrated suspension in step (2), a water soluble stabilizer, an antioxidant and a preservative are added with water to obtain the lipid hydrated suspension; or a water soluble stabilizer, an antioxidant and a preservative are added with an aqueous solution comprising a water soluble softener to obtain the lipid hydrated suspension.

16. The method according to any one of claims 9-14, wherein the organic solvent used is one or more selected from the group consisting of dichloromethane, chloroform, diethyl ether and methanol.

Curves of the accumulated amount of penetration
between two flexible liposomes

F i g. 1

# INTERNATIONAL SEARCH REPORT

| International application No. |
| --- |
| PCT/CN2010/071011 |

**A. CLASSIFICATION OF SUBJECT MATTER**

See the extra sheet
According to International Patent Classification (IPC) or to both national classification and IPC

**B. FIELDS SEARCHED**

Minimum documentation searched (classification system followed by classification symbols)

IPC: A61K, A61P, A61Q

Documentation searched other than minimum documentation to the extent that such documents are included in the fields searched

Electronic data base consulted during the international search (name of data base and, where practicable, search terms used)

DATABASE: WPI, EPODOC, CNPAT(CN), CNKI(CN), CA, MEDLINE, EMBASE;

SEARCH TERMS: resveratrol, liposome+, 3,4,5-trihydrolystilbence, flexible liposome+, transfersome+, delivery system, ethosome+, surfactant, surface active agent, flexible agent, phospholipid, phosphatidylcholine, polysorbate, tween, cholic acid, sodium cholate, span, propylene glycol, ethanol

**C. DOCUMENTS CONSIDERED TO BE RELEVANT**

| Category* | Citation of document, with indication, where appropriate, of the relevant passages | Relevant to claim No. |
| --- | --- | --- |
| X | CN 1951368 A (LI Wanzhong), 25 April 2007 (25.04.2007), see claims 4, 6 | 1-16 |
| A | CN 101317819 A (TIANJIN INSTITUTE OF PHARMACEUTICAL RESEARCH), 10 December 2008 (10.12.2008), see the whole document | 1-16 |
| A | CN 1794967 A (ENPRANI CO. LTD.), 28 June 2006 (28.06.2006), see the whole document | 1-16 |
| A | CN 101170989 A (RECKITT BENCKISER (UK) LTD.), 30 April 2008 (30.04.2008), see the whole document | 1-16 |
| A | CN 1327380 A (HINDUSTAN LEVER LTD.), 19 December 2001 (19.12.2001), see the whole document | 1-16 |

☐ Further documents are listed in the continuation of Box C.   ☒ See patent family annex.

| | |
| --- | --- |
| *     Special categories of cited documents:<br>"A"   document defining the general state of the art which is not considered to be of particular relevance<br>"E"   earlier application or patent but published on or after the international filing date<br>"L"   document which may throw doubts on priority claim (S) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"document member of the same patent family |

| Date of the actual completion of the international search | Date of mailing of the international search report |
| --- | --- |
| 31 May 2010(31.05.2010) | **17 Jun. 2010 (17.06.2010)** |

| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>**LIANG Jingchen**<br>Telephone No. (86-10)62411134 |

Form PCT/ISA /210 (second sheet) (July 2009)

## INTERNATIONAL SEARCH REPORT
### Information on patent family members

| | International application No. |
|---|---|
| | PCT/CN2010/071011 |

| Patent Documents referred in the Report | Publication Date | Patent Family | Publication Date |
|---|---|---|---|
| CN 1951368 A | 25.04.2007 | CN 100453071 C | 21.01.2009 |
| CN 101317819 A | 10.12.2008 | None | |
| CN 1794967 A | 28.06.2006 | WO 2004103265 A2 | 02.12.2004 |
| | | KR 20040101665 A | 03.12.2004 |
| | | EP 1626702 A2 | 22.02.2006 |
| | | KR 20050039927 A | 03.05.2005 |
| | | KR 100521768 B | 17.10.2005 |
| | | JP 2006528239 T | 14.12.2006 |
| | | US 2007009455 A1 | 11.01.2007 |
| | | KR 100553157 B1 | 12.04.2007 |
| CN 101170989 A | 30.04.2008 | EP 1726292 A1 | 29.11.2006 |
| | | WO 2006125981 A1 | 30.11.2006 |
| | | AU 2006250994 A1 | 30.11.2006 |
| | | INCHENP 200705929 E | 27.06.2008 |
| | | CA 2608031 A1 | 30.11.2006 |
| | | JP 2008542251 T | 27.11.2008 |
| | | ZA 200708212 A | 31.12.2008 |
| | | US 2009082473 A1 | 26.03.2009 |
| CN 1327380 A | 19.12.2001 | WO 9904747 A2 | 04.02.1999 |
| | | AU 8858498 A | 16.02.1999 |
| | | EP 0980235 A2 | 23.02.2000 |
| | | ZA 9806039 A | 29.03.2000 |
| | | CZ 20000283 A3 | 12.07.2000 |
| | | BR 9810810 A | 12.09.2000 |
| | | AU 730825 B | 15.03.2001 |
| | | JP 2001510777 T | 07.08.2001 |
| | | KR 20010022170 A | 15.03.2001 |
| | | MXPA 00000493 A | 01.11.2000 |

Form PCT/ISA /210 (patent family annex) (July 2009)

## INTERNATIONAL SEARCH REPORT
Information on patent family members

| International application No. |
|---|
| PCT/CN2010/071011 |

| Patent Documents referred in the Report | Publication Date | Patent Family | Publication Date |
|---|---|---|---|
| | | TW 480178 A | 21.03.2002 |
| | | EP 0980235 B1 | 28.05.2003 |
| | | RU 2203036 C2 | 27.04.2003 |
| | | DE 69815099 E | 03.07.2003 |
| | | MX 214214 B | 13.05.2003 |
| | | ES 2200362 T3 | 01.03.2004 |
| | | CZ 295608 B6 | 17.08.2005 |
| | | CN 1162145 C | 18.08.2004 |
| | | KR 100514271 B | 13.09.2005 |
| | | CA 2299458 C | 26.02.2008 |
| | | IN 190488 B | 02.08.2003 |

Form PCT/ISA /210 (patent family annex) (July 2009)

## INTERNATIONAL SEARCH REPORT

International application No.

PCT/CN2010/071011

Continuation of classification of subject matter:

A61K 9/127 (2006. 01) i

A61K 31/05 (2006. 01) i

A61K8/14 (2006. 01) i

A61P17/18 (2006. 01) i

A61P17/10 (2006. 01) i

A61Q19/08 (2006. 01) i

Form PCT/ISA /210 (extra sheet) (July 2009)